# EUROPÄISCHE PATENTSCHRIFT

(11) **EP 2 538 775 B1**
(45) Veröffentlichungstag und Bekanntmachung des Hinweises auf die Patenterteilung: **07.01.2015**
(21) Anmeldenummer: 11704736.5
(22) Anmeldetag: 22.02.2011
(51) Int. Cl.: A01K 67/033, A01P 7/04

(54) **AUSBRINGSYSTEM FÜR DIE AUSBRINGUNG MEHRERER NUETZLINGS-VERMEHRUNGSKOLONIEN IN EINEN LAND- BZW. GARTENWIRTSCHAFTLICHEN BESTAND**
SYSTEM FOR THE DISTRIBUTION OF A PLURALITY OF COLONIES OF BENEFICIAL INSECTS IN AN AGRICULTURALLY OR HORTICULTURALLY USED AREA
SYSTÈME DE DISTRIBUTION D'UNE PLURALITÉ DES COLONIES D'INSECTES BÉNÉFIQUES DANS UNE ZONE AGRICULTURELLE OU HORTICULTURELLE

(30) Priorität: 25.02.2010 DE 102010009342
(43) Veröffentlichungstag der Anmeldung: 02.01.2013
(73) Patentinhaber: Biocare Gesellschaft für Biologische Schutzmittel mbH, 37574 Einbeck (DE)
(72) Erfinder: BEITZEN-HEINEKE, Wilhelm, 37574 Einbeck (DE); BILGESHAUSEN, Ulli, 37081 Göttingen (DE)
(74) Vertreter: Grättinger Möhring von Poschinger Patentanwälte Partnerschaft
(86) Internationale Anmeldenummer: PCT/EP2011/000843
(87) Internationale Veröffentlichungsnummer: WO 2011/104002

(56) Entgegenhaltungen:
- EP-A1- 1 161 863
- EP-A1- 2 042 036
- AU-B3- 648 244
- DE-A1- 4 421 366
- US-A1- 2005 178 337
- US-A1- 2009 094 890
- AMW-Nuetzlinge: "Die TrichoKarte MAIS und die Trichokugel MAIS zur biologischen Bekämpfung des Maiszünslers", , 23. April 2009 (2009-04-23), XP002638102, Gefunden im Internet: URL:http://replay.web.archive.org/20090423 011331/http://www.amw-nuetzlinge.de/images /media/MAISPROSPEKT_2008.pdf [gefunden am 2011-05-19]

## Beschreibung

Die vorliegende Erfindung liegt auf dem Gebiet des biologischen Pflanzenschutzes. Sie betrifft ein Ausbringsystem für die Ausbringung mehrerer Nützlings-Vermehrungskolonien in einen land- bzw. gartenwirtschaftlichen Bestand, wobei als eine Nützlings-Vermehrungskolonie jeweils ein biologisches System umfassend eine vermehrungsfähige Population von Nützlingen, eine der Ernährung der Nützlinge während ihrer Entwicklung dienende vermehrungsfähige Population von Wirten und einen der Ernährung der Wirte dienenden Nahrungsvorrat umfasst.

Es ist bekannt, Schadinsekten, welche auf Garten- und landwirtschaftlichen Anbauflächen Schäden verursachen, durch Einsatz von Nützlingen dergestalt zu bekämpfen, dass die Nützlinge (in zumindest einem Entwicklungsstadium) die Schädlinge (in mindestens einem Entwicklungsstadium) parasitieren, d.h. zur eigenen Vermehrung oder als Futter nutzen. Bekannte Nützlinge für verschiedene in Europa weit verbreitete Schädlinge sind insbesondere verschiedene Raubmilben und Schlupfwespen.

Ein erfolgreicher Einsatz von Nützlingen in Kulturen, d.h. auf in land- und/oder gartenwirtschaftlichen Beständen ist von der erfolgreichen Etablierung der Nützlinge abhängig. Je früher die Nützlinge in dem jeweiligen Bestand vorhanden sind, desto geringer ist die Gefahr, dass sich Schädlinge in den Beständen etablieren und dort Schaden anrichten können. Ein Ausbringsystem mit einer verlängerten Freisetzungszeit (controlled release) erlaubt einen früheren Einsatztermin und steigert daher den Erfolg einer solchen Maßnahme der biologischen Schädlingsbekämpfung maßgeblich. Vor diesem Hintergrund ist im Interesse einer möglichst effizienten Anwendung von Verfahren zum biologischen Pflanzenschutz bekannt, die Nützlinge auf die betreffenden Anbauflächen in Einheiten der eingangs angegebenen Art auszubringen, welche Vermehrungs-, Brut- und Aufzuchtstätten darstellen, wobei die Nützlinge in diesem Fall Teil einer Nützlings-Vermehrungskolonie in dem einleitend dargestellten Sinne sind. Denn auf diese Weise werden über einen längeren Zeitraum im behandelten land- bzw. gartenwirtschaftlichen Bestand selbst Nützlinge (in den für die Schädlingsbekämpfung maßgeblichen Entwicklungsstadien) bereitgestellt, so dass die Anwendungshäufigkeit solcher Ausbringeinheiten gegenüber der Ausbringung von lediglich Nützlinge enthaltenden Ausbringeinheiten reduziert werden kann. Dadurch, dass sich die Nützlinge nach ihrem Ausbringen weiter vermehren können, ergeben sich erhebliche Kostenvorteile. Weiterhin ist eine Vorverlegung des Applikationszeitpunktes der Nützlinge möglich. In manchen Fällen tritt hinwiederum der Schädlingsbefall später auf als erwartet. In einem solchen Fall finden die Nützlinge, sofern sie nicht mit einer Futter enthaltenden Ausbringeinheit ausgebracht werden, kein Futter, können sich nicht vermehren oder verhungern sogar. Die Ausbringung der Nützlinge in einer Futter enthaltenden Vermehrungs-, Brut- und Aufzuchtstätte sichert daher den Erfolg der eingesetzten Nützlinge, die sich über einen längeren Zeitraum in der Vermehrungs-, Brut- und Aufzuchtstätte weiterentwickeln können.

Solche eine Vermehrungs-, Brut- und Aufzuchtstätte darstellenden, eine Nützlings-Vermehrungskolonie beinhaltenden Ausbringeinheiten können dabei beispielsweise als Nützlinge eine vermehrungsfähige Population von Raubmilben, als Wirte eine vermehrungsfähige Population von Opfermilben und als Nahrungsvorrat für die Wirte eine Mischung aus verschiedenen biogenen Substanzen wie Getreideprodukte oder ähnliches enthalten. Dergleichen ist insbesondere beschrieben in der WO 2006/057552 A1 und der WO 2008/015393 A2, die insoweit einen maßgeblichen Stand der Technik darstellen. Hinzuweisen ist in diesem Zusammenhang darauf, dass der Begriff "Wirt" im Rahmen der vorliegenden Anmeldung im weiteren Sinne zu verstehen ist; er erfasst nicht nur Wirte im engeren Sinne, die durch die Nützlinge in einem von deren Entwicklungsstadien parasitiert werden, sondern auch solche Lebewesen, die von den Nützlingen in einem von deren Entwicklungsstadien verzehrtes Futter produzieren, z.B. in Form von Eiern oder aber auch Stoffwechselprodukten.

Nach der WO 2008/015393 A2 können solche der Ausbringung einer Nützlings-Vermehrungskolonie in einen land- bzw. gartenwirtschaftlichen Bestand dienenden, Vermehrungs-, Brut- und Aufzuchtstätten bildenden Ausbringeinheiten, ohne dass dies im Einzelnen erläutert wird, in Form von Töpfen, Flaschen, Schachteln, Kartons, Taschen, Röhren, Säckchen und Kissen ausgeführt sein. Durch offenkundige Vorbenutzung sind weiterhin Bänder bekannt geworden, in denen in bestimmten Abständen entsprechende Nützling-/Wirt-Populationen samt Nahrungsvorrat für die Wirte untergebracht sind. Die entsprechenden Bänder werden auf Rollen zu jeweils beispielsweise 50 m oder 100 m aufgerollt vertrieben. Der Anwender legt das von der Rolle abgewickelte Band längs der in Gewächshäusern aufgestellten Pflanzentische aus.

Die EP 2042036 A1 befasst sich mit der Bereitstellung einer vermehrungsfähigen Population der Schlupfwespe Amblyseius swirskii zum Einsatz in der biologischen Schädlingsbekämpfung in der Landwirtschaft. Die künstliche Wirtspopulation, welche in der entsprechenden Nützlings-Vermehrungskolonie der Ernährung der Schlupfwespen Amblyseius swirskii dient, umfasst dabei Astigmatina Milben. Die Nützlings-Vermehrungskolonie ist in einem Behältnis untergebracht, welches in dem landwirtschaftlichen Bestand auf dem Boden ausgelegt oder aber aufgehängt wird. Um es aufhängen zu können, kann das entsprechende Behältnis einen Haken oder eine Schnur aufweisen.

Zwei unterschiedliche Ausbringeinheiten für in der biologischen Schädlingsbekämpfung eingesetzte Schlupfwespen sind in der Firmenbroschüre "Die TrichKarte MAIS - Die TrichoKugel MAIS" der AMW Nützlings GmbH erläutert, nämlich einerseits die an Pflanzen anzuhängenden Kartonträger "TrichoKarte" und andererseits die (manuell oder maschinell) auszuwerfenden Ausbringkugeln "TrichoKugel". Mit beiden Ausbringeinheiten befasst sich auch die EP 1161863 A1, wobei nach diesem Dokument die (primär im Hinblick auf die magazinierte Ausbringung, beispielsweise mittels Kleinflugzeugen gestalteten) Ausbringkugeln zu ihrer Einzelaufhängung an Sträuchern oder Bäumen optional auch mit einer Aufhängung versehen sein können. Unter Verwendung jener ringförmigen Aufhängungen können auch mehrere Ausbringkugeln zu einem wabenförmigen Verband miteinander verbunden werden.

Die vorliegende Erfindung hat sich zur Aufgabe gemacht, die Effizienz der biologischen Schädlingsbekämpfung durch Ausbringung von Nützlings-Vermehrungskolonien in einen land- bzw. gartenwirtschaftlichen Bestand gegenüber dem vorstehend dargelegten Stand der Technik in verschiedener Hinsicht zu steigern sowie eine größere Flexibilität hinsichtlich der Anwendungsmöglichkeiten zu erreichen.

Zur Lösung dieser Aufgabenstellung umfasst nach der vorliegenden Erfindung ein Ausbringsystem für die Ausbringung mehrerer Nützlings-Vermehrungskolonien in einen land- bzw. gartenwirtschaftlichen Bestand eine Mehrzahl von der Ausbringung jeweils einer Nützlings-Vermehrungskolonie in einen land- oder gartenwirtschaftlichen Bestand dienenden Ausbringeinheiten und weist weiterhin die folgenden Merkmale auf:
die Ausbringeinheiten umfassen jeweils eine Vermehrungs-,
Brut- und Aufzuchtkugel für die Nützlinge;
die Vermehrungs-, Brut- und Aufzuchtkugeln enthalten jeweils eine Nützlings-/Wirt-Population umfassend eine vermehrungsfähige Population von Nützlingen sowie eine vermehrungsfähige Population von Wirten;
die Vermehrungs-, Brut- und Aufzuchtkugeln enthalten weiterhin jeweils einen Nahrungsvorrat für die Wirte;
sie bestehen aus einem biologisch abbaubaren Material;
die Ausbringeinheiten sind mittels Verbindungsschnüren miteinander verbunden.

Eines der zentralen, kombinatorisch zusammenwirkenden Merkmale des erfindungsgemäßen Ausbringsystems besteht demgemäß darin, dass es mehrere - über Verbindungsschnüre miteinander verbundene - Vermehrungs-, Brut- und Aufzuchtkugeln für die Nützlinge, d.h. kugelförmige Vermehrungs-, Brut- und Aufzuchtstätten für die Nützlinge aufweist. Dies schlägt sich in durchaus überraschender Weise in einer erheblichen Anzahl von signifikanten Vorteilen nieder, die zu einer maßgeblichen Steigerung sowohl der Effizienz der biologischen Schädlingsbekämpfung als auch der Flexibilität hinsichtlich der Anwendungsmöglichkeiten beitragen.

So wird namentlich der Anwendungsbereich für die biologische Schädlingsbekämpfung in einem land- bzw. gartenwirtschaftlichen Bestand unter Verwendung von Vermehrungs-, Brut- und Aufzuchtstätten für Nützlinge bildenden, eine Nützlings-Vermehrungskolonie beinhaltenden Ausbringeinheiten der hier in Rede stehenden Art in verschiedene Freilandanwendungen hinein ausgedehnt, für die dies bisher nicht mit hinreichender Effizienz möglich war. Insoweit kommt zum Tragen, dass das spezifische, besonders geringe Oberflächen-/VolumenVerhältnis der erfindungsgemäß eingesetzten kugelförmigen Vermehrungs-, Brut- und Aufzuchtstätten dazu beiträgt, dass tageszeitliche Schwankungen der Temperatur in der Umgebung, in der die erfindungsgemäßen Ausbringeinheiten eingesetzt werden, sich auf die im Inneren der Vermehrungs-, Brut- und Aufzuchtkugel bestehenden Bedingungen nur geringstmöglich auswirken. Insoweit besteht, insbesondere wenn auch das für die Herstellung der Vermehrungs-, Brut- und Aufzuchtkugeln verwendete Material und deren Wandstärke entsprechend abgestimmt sind (s.u.), im Inneren der Vermehrungs-, Brut- und Aufzuchtkugeln ein die Vermehrung der Nützlinge günstig beeinflussendes vergleichsweise ausgeglichenes Klima.

Die im Rahmen der vorliegenden Erfindung zum Einsatz kommenden Vermehrungs-, Brut- und Aufzuchtkugeln bestehen aus einem biologisch abbaubaren Material, besonders bevorzugt auf Zellstoffbasis. Ein auf Zellstoffbasis hergestelltes Material wirkt sich durch hervorragende klimaausgleichende Eigenschaften äußerst günstig auf das im Inneren der Vermehrungs-, Brut- und Aufzuchtkugeln bestehende Klima aus, was wiederum der Vermehrungs- und Überlebensrate sowohl der Nützlinge als auch der Wirte zugute kommt und weiterhin eine witterungsbedingte Beeinträchtigung der Qualität des Nahrungsvorrats für die Wirte verhindert. Bei Vermehrungs-, Brut- und Aufzuchtkugeln mit typischen Außenabmessungen, d.h. einem Durchmesser von etwa 1,5cm bis 2,5cm, sind in dieser Hinsicht Wandstärken der Vermehrungs-, Brut- und Aufzuchtkugeln von zwischen etwa 0,5mm und 2mm, insbesondere zwischen etwa 0,8mm und 1,5mm besonders vorteilhaft. Bei solchen Wandstärken besteht ausreichend Masse als temperaturausgleichender Wärmespeicher zur Abpufferung der in typischen Freilandanwendungen zu erwartenden Temperaturunterschiede zwischen Tag und Nacht, ohne dass unnötig viel Material verwendet wird; und zugleich ist diese Wandstärke für einen effizienten Luftfeuchtigkeitsausgleich günstig, was sich angesichts dessen, dass hohe Luftfeuchten für die Nützlingsvermehrung bedeutsam sind, positiv auf die Vermehrungsrate und somit auf die Effizienz der biologischen Schädlingsbekämpfung auswirkt. Als Schutz vor zu viel Feuchtigkeit, insbesondere vor Nässe, weisen die Vermehrungs-, Brut- und Aufzuchtkugeln besonders bevorzugt eine wasserabweisende Beschichtung, insbesondere aus Paraffin, auf; oder die Vermehrungs-, Brut- und Aufzuchtkugeln bestehen aus in Paraffin getränktem Zellstoff. Letzteres führt zu besonders günstigen Eigenschaften hinsichtlich des Mikroklimas in der Vermehrungs-, Brut- und Aufzuchtstätte. Denn einerseits perlt auf die Oberfläche der Ausbringeinheit auftreffendes Wasser (Beregnung, natürlicher Regen) ab; andererseits wird Wasser, freilich ohne Schaden anzurichten, in gewissem Umfang kapillar gespeichert und anschließend wieder abgegeben, wobei die Wasserspeicherkapazität durch unterschiedliche Paraffinmengen an den jeweiligen Anwendungszweck angepasst werden kann. Insbesondere in warmen bzw. heißen Umgebungen wirkt sich die hierdurch (namentlich bei Sonneneinstrahlung) entstehende Verdunstungskälte und die dadurch erfolgende Temperierung (Kühlung) der Ausbringeinheiten positiv auf die Vermehrung der Nützlinge aus. Auch für die Wirkung dieses oberflächlichen Kühleffekts auf das Innenraumklima der Vermehrungs-, Brut- und Aufzuchtkugeln ist unter Berücksichtigung der Eigenschaften insbesondere von Zellulosematerial die aufgezeigte Wandstärke zwischen etwa 0,5mm und 2mm, insbesondere zwischen etwa 0,8mm und 1,5mm günstig.

Ähnlich gute Eigenschaften im Hinblick auf das in den Vermehrungs-, Brut- und Aufzuchtkugeln bestehende, die Entwicklung der Nützlinge günstig beeinflussende Mikroklima ergeben sich im Falle der Herstellung der Vermehrungs-, Brut- und Aufzuchtkugeln aus extrudierter Maisstärke, allerdings bei einer - zumindest bei Verwendung konventioneller, nicht modifizierter Stärke - gegenüber Zellstoff als Ausgangsmaterial reduzierten biologischen Abbaubarkeit.

Weiterhin besteht ein die Effizienz, d.h. die Kosten der Anwendung, positiv beeinflussender Faktor in einem vergleichsweise geringen Transportaufwand für die erfindungsgemäßen Ausbringsysteme. Denn während nach dem Stand der Technik ein relativ hoher Transportaufwand entsteht, weil typischerweise etwa 75% des auf der entsprechenden Rolle aufgewickelten bandförmigen Materials einen im Hinblick auf die Zweckbestimmung unwirksamen Abfall darstellen, besteht bei den erfindungsgemäßen Ausbringsystemen nahezu kein nicht wirksamer Abfall. Und weiterhin lassen sich die kugelförmigen Ausbringeinheiten der erfindungsgemäßen Ausbringsysteme beim Versand und beim Weg zur Anwendung mit sehr hoher Dichte packen. Der Transportaufwand ist auf diese Weise in doppelter Hinsicht minimal; und es fällt, anders als dies für die bekannten Bänder gilt, im land- bzw. gartenwirtschaftlichen Bestand kaum unnötiger Abfall an.

Die erfindungsgemäß eingesetzten, vorstehend erläuterten Ausbringeinheiten sind, gemäß einem weiteren wesentlichen Aspekt der vorliegenden Erfindung, zur Bildung eines mehrere Ausbringkugeln umfassenden Ausbringsystems (zumindest zunächst) zu mehreren jeweils mittels Verbindungsschnüren miteinander verbunden; auf diese Weise wird ein eine Mehrzahl von mittels Verbindungsschnüren miteinander verbundenen Vermehrungs-, Brut- und Aufzuchtkugeln umfassendes perlenschnurähnliches Ausbringsystem für die Ausbringung von Nützlingen, insbesondere Raubmilben, im biologischen Pflanzenschutz gebildet. Hierdurch erweitern sich das Anwendungsspektrum und die Anwendungs- bzw. Ausbringungsmöglichkeiten von Nützlings-Vermehrungskolonien ganz erheblich gegenüber dem Stand der Technik. Denn die Ausbringeinheiten können auf einfachste Weise gruppenweise ausgebracht werden. So kann beispielsweise bei einem eine Vielzahl von Ausbringeinheiten, die mittels (typischerweise eine gegenüber dem Durchmesser der Vermehrungs-, Brut- und Aufzuchtkugeln wesentlich kleinere Dicke und/oder gegenüber der Querschnittsfläche der Vermehrungs-, Brut- und Aufzuchtkugeln wesentlich kleinere Querschnittsfläche aufweisenden) Verbindungsschnüren miteinander verbunden und jeweils als Vermehrungs-, Brut- und Aufzuchtkugeln ausgeführt sind, umfassenden Ausgangsmaterial (z.B. ein 100 m langes System mit 200 im Abstand von jeweils 50 cm zueinander angeordneten Vermehrungs-, Brut- und Aufzuchtkugeln) immer (nur) jede zweite Verbindungsschnur durchtrennt werden, so dass als den spezifischen Bedürfnissen angepasstes Ausbringsystem jeweils ein Paar von über eine Verbindungsschnur miteinander verbundener Vermehrungs-, Brut- und Aufzuchtkugeln entsteht. Solche Gebilde können beispielsweise einfach über Blattansätze oder dergl. gelegt bzw. gehängt werden und bleiben dort trotzdem relativ zuverlässig hängen, auch bei ungünstigen Witterungsverhältnissen (Wind), was auch zur Applikation von Nützlingen auf Bäumen hilfreich sein kann. Sie können auch (ggf. maschinell vom Boden aus und/oder aus der Luft) ausgeworfen werden, wobei sie typischerweise an den Pflanzen des Bestandes - in mehr oder weniger idealer Anwendungshöhe über dem Boden - hängen bleiben, d.h. nicht auf den Boden fallen; eine für typische Anwendungsfälle zweckmäßige Ausgestaltung in dieser Hinsicht liegt vor, wenn die Länge der Verbindungsschnur jeweils etwa dem 5-fachen bis 50-fachen, besonders bevorzugt etwa dem 10-bis 20-fachen Wert des Durchmessers der Vermehrungs-, Brut- und Aufzuchtkugeln entspricht, wobei angesichts dessen, dass einzelne Anwendungen von Nützlingen bei bestimmten Kulturen eine möglichst nahe Applikation am Wirkungsort der Nützlinge erfordern, im Einzelfall durchaus Abweichungen von dieser Dimensionierung der Länge der Verbindungsschnüre zweckmäßig sind. Solche eine Mehrzahl von miteinander mittels Verbindungsschnüren verbundene Vermehrungs-, Brut- und Aufzuchtkugeln umfassenden Gebilde (Ausbringsysteme) sind demnach insbesondere in solchen Anwendungen mit Vorteil einsetzbar, in denen eine Ablage der Ausbringeinheiten auf dem Boden des behandelten land- bzw. gartenwirtschaftlichen Bestands unerwünscht ist.

Gegenüber dem weiter oben dargelegten Stand der Technik ist festzustellen, dass sich die vorstehend erläuterten perlenschnurartigen, eine Vielzahl von mittels Verbindungsschnüren miteinander verbundene Vermehrungs-, Brut- und Aufzuchtkugeln umfassende Ausbringsysteme deutlich raumsparender für den Transport verpacken lassen als ein aufgewickeltes Band mit Tüten.

Werden die zunächst zu einem perlenschnurähnlichen Ausbringsystem miteinander verbundenen Vermehrungs-, Brut- und Aufzuchtkugeln durch Durchtrennen sämtlicher Verbindungsschnüre vollständig vereinzelt, können die beiden Verbindungsschnurenden zur individuellen Anbringung der jeweiligen einzelnen Ausbringeinheit an Pflanzen, Pfosten, Drähten oder dergl. verwendet werden. Für andere Anwendungen kommt in Betracht, das perlenschnurähnliche Ausbringsystem über oder in dem garten- bzw. landwirtschaftlichen Bestand zu spannen. Erkennbar ergeben sich somit diverse unterschiedliche, auf die spezifischen Anforderungen eingehenden Anwendungs- und Ausbringmöglichkeiten, was zu einer bisher nicht bekannten Flexibilität in der biologischen Schädlingsbekämpfung unter Verwendung von solchen Vermehrungs-, Brut- und Aufzuchtstätten führt, die eine Nützlings-Vermehrungskolonie, d.h. neben einer Population von Nützlingen auch eine Population von Wirten und einen Nahrungsvorrat für die Wirte, umfassen.

Bei einer Anwendung vereinzelter Ausbringeinheiten der Ausbringsysteme nach der vorliegenden Erfindung kommt im Sinne einer maximalen Effizienz weiterhin zum Tragen, dass bei optimierter Ausbringung eine kleinstmögliche Anzahl von Ausbringeinheiten ausreichend ist, jedenfalls eine geringere Anzahl als im Falle der bekannten Bänder. Denn bei letzteren bedarf es einer deutlich höheren als der rechnerisch ermittelbaren Anzahl von Nützlings-/Wirts-Populationen, was damit zusammenhängt, dass die die jeweilige Brut- und Vermehrungsstätte verlassenden Nützlinge sich typischerweise etwa kugelförmig um letztere herum ausbreiten. Um systematische nützlingsfreie Räume zwischen zwei ausgelegten Bändern mit jeweils im Abstand zueinander angeordneten Brut- und Vermehrungsstätten zu vermeiden, müssen die besagten Bänder vergleichsweise dicht ausgelegt werden.

Die im Rahmen der vorliegenden Erfindung eingesetzten Vermehrungs-, Brut- und Aufzuchtkugeln besitzen bevorzugt auf jeder Hälfte zwei Austrittsöffnungen zum Luftaustausch (sowohl die Nützlinge als auch die Wirte produzieren CO₂) und zum Auswandern der Nützlinge in den land- bzw. gartenwirtschaftlichen Bestand. Dabei ist bevorzugt jeweils eines der beiden Löcher von innen nach außen und das andere Loch von außen nach innen gestanzt; auf diese Weise wird sichergestellt, dass je eine Seite eine Austrittsöffnung ohne inneren Stanzgrat, der möglicherweise das Auswandern der Nützlinge erschwert bzw. behindert, aufweist.

Weiterhin ist für eine maximale Effizienz auch im Sinne einer besonders lang anhaltenden Wirkung und geringen Anwendungsfrequenz der Ausbringeinheiten besonders vorteilhaft, wenn die Nützlingspopulation der einzelnen Ausbringeinheiten Nützlinge und/oder die Wirtspopulation der einzelnen Ausbringeinheiten Wirte in verschiedenen Entwicklungsstadien umfasst.

Die bei den erfindungsgemäßen Ausbringsystemen eingesetzten flexiblen Verbindungsschnüre bestehen besonders bevorzugt aus einem biologisch abbaubaren Material. Ihre Reißfestigkeit liegt besonders bevorzugt zwischen dem 50-fachen und dem 500-fachen Wert der dem Gewicht einer Vermehrungs-, Brut- und Aufzuchtkugel entsprechenden Gewichtskraft. In diesem Falle sind sie einerseits für alle denkbaren Anwendungen ausreichend fest und andererseits trotzdem typischerweise selbst von Hand leicht zu durchtrennen, was für eine flexible, individuelle Anwendung im Bestand von großem Vorteil ist. Die Gestaltung der Verbindungsschnüre unterliegt im Übrigen keiner Beschränkung. So können im Rahmen der vorliegenden Erfindung insbesondere Verbindungsschnüre unterschiedlicher Querschnittsformen Verwendung finden, z.B. runde Schnüre oder auch Flachschnüre. Die jeweils zwei Vermehrungs-, Brut- und Aufzuchtkugeln miteinander verbindenden Verbindungsschnüre können dabei weiterhin auch ineinander übergehen im Sinne der Bildung einer durchgehenden, sich über die Länge des Ausbringsystems erstreckenden Schnur, an der die einzelnen Vermehrungs-, Brut- und Aufzuchtkugeln in bestimmten Abständen angebracht sind, z.B. durch Kleben. Ist eine solche durchgehende Schnur mit einem flachen Querschnitt vorgesehen, so kann diese in Längsrichtung dachförmig gefaltet sein. Dies bringt zwei Vorteile mit sich, nämlich eine zuverlässigere Anbringung der Vermehrungs-, Brut- und Aufzuchtkugeln an der Schnur jeweils über zwei Klebepunkte und einen (je nach der Breite der Schnur mehr oder weniger ausgeprägten) Schutz der Vermehrungs-, Brut- und Aufzuchtkugeln vor Witterungseinflüssen. Weiterhin kommt in Betracht, dass mehrere solcher durchgehenden Schnüre vorgesehen sind, an denen die Vermehrungs-, Brut- und Aufzuchtkugeln angebracht sind. Dies ist von Vorteil im Hinblick auf eine besonders zuverlässige Fixierung der Vermehrungs-, Brut- und Aufzuchtkugeln, und zwar insbesondere auch während des Ausbringens des betreffenden Ausbringsystems; denn auf diese Weise wird die Gefahr, dass Vermehrungs-, Brut- und Aufzuchtkugeln abgerissen werden, maßgeblich reduziert, verglichen mit solchen Ausbringsystemen, bei denen die Vermehrungs-, Brut- und Aufzuchtkugeln an nur einer Schnur angebracht sind.

Gemäß einer besonders bevorzugten Weiterbildung sind bei dem erfindungsgemäßen, eine Mehrzahl von mittels Verbindungsschnüren miteinander verbundenen Vermehrungs-, Brut- und Aufzuchtkugeln umfassenden perlenschnurähnlichen Ausbringsystem die Verbindungsschnüre durch einen - eine durchgehende Schnur in dem weiter oben dargelegten Sinn darstellenden - Schlauch gebildet. Dieser ist in einem solchen Maße aufweitbar, dass die Vermehrungs-, Brut- und Aufzuchtkugeln in ihm aufgenommen werden können. Beispielsweise kann dar betreffende Schlauch gewirkt, gestrickt oder geflochten sein, bevorzugt unter Verwendung von ihrerseits zumindest in einem gewissen Maß dehnbaren Fäden. Die Vermehrungs-, Brut- und Aufzuchtkugeln sind mit Abstand zueinander in dem Schlauch aufgenommen, der im Bereich der Vermehrungs-, Brut- und Aufzuchtkugeln in dem hierzu erforderlichen Maß geweitet ist, während zwischen jeweils zwei einander benachbarten Vermehrungs-, Brut- und Aufzuchtkugeln der Schlauch im Sinne einer Schnur dünn und flexibel ist. Durch die Aufweitung des Schlauches im Bereich der Vermehrungs-, Brut- und Aufzuchtkugeln und den hierdurch erreichten entsprechend großen Abstand der Einzelfäden das Schlauches zueinander ist mit ausreichender Zuverlässigkeit sichergestellt, dass zumindest ein Teil der Austrittsöffnungen nicht durch Einzelfäden des Schlauches verlegt sind und die Nützlinge die Vermehrungs-, Brut- und Aufzuchtkugeln ungehindert verlassen können. Eine solche Weiterbildung des Ausbringsystems zeichnet sich bei einer vergleichsweise einfachen, kostengünstigen Herstellung durch eine äußerst hohe Zuverlässigkeit der Verbindung der einzelnen Vermehrungs-, Brut- und Aufzuchtkugeln untereinander, auch während des Ausbringens des betreffenden Ausbringsystems, aus.

Im Folgenden wird die vorliegende Erfindung anhand von in der Zeichnung veranschaulichten Ausführungsbeispielen näher erläutert. Dabei zeigt
- Fig. 1: eine bei einem bevorzugten erfindungsgemäßen Ausbringsystem verwendete Ausbringeinheit in teilweise geschnittener Darstellung,
- Fig. 2: ein zwei mittels einer Verbindungsschnur miteinander verbundene Ausbringeinheiten nach Fig. 1 umfassendes Ausbringsystem,
- Fig. 3: ein Sonderform eines mittels einer Verbindungsschnur miteinander verbundene Ausbringeinheiten nach Fig. 1 umfassenden Ausbringsystems und
- Fig. 4: ein weiteres Anwendungsbeispiel für ein Ausbringsystem nach der vorliegenden Erfindung.

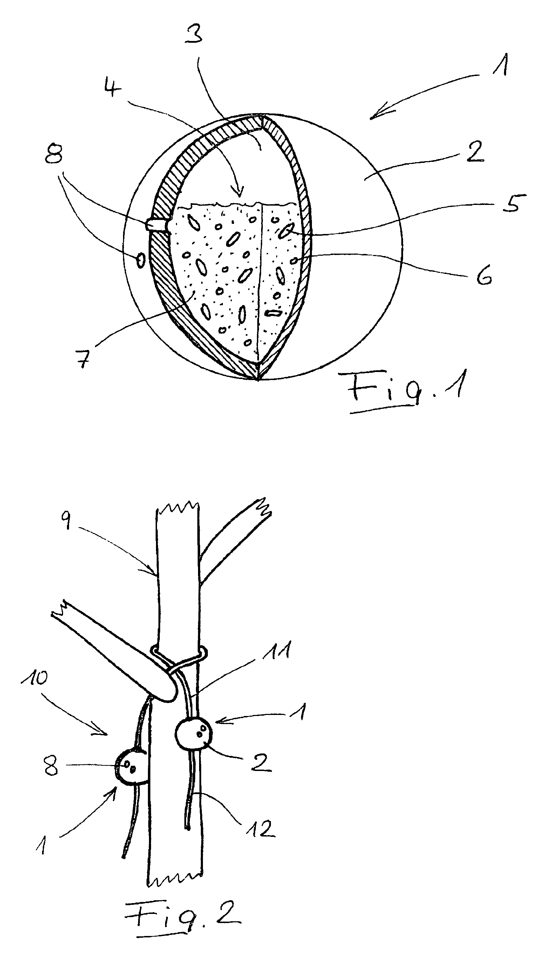

Die in Fig. 1 (nicht maßstabsgerecht) gezeigte Ausbringeinheit 1 für eine im Rahmen des biologischen Pflanzenschutzes erfolgende Ausbringung von Nützlingen in einen land- oder gartenwirtschaftlichen Bestand umfasst eine Vermehrungs-, Brut- und Aufzuchtkugel 2. Diese besteht aus Zellstoffmaterial und weist eine Wandstärke von 1mm auf; das Zellstoffmaterial ist in Paraffin getränkt, so dass es sowohl oberflächlich wasserabweisende Eigenschaften aufweist als auch einen kapillaren Wasserspeicher bildet. Der Durchmesser Vermehrungs-, Brut- und Aufzuchtkugel 2 beträgt 2cm.

In dem Inneren der Vermehrungs-, Brut- und Aufzuchtkugel 2, d.h. dem Hohlraum 3 befindet sich eine Nützlings-Vermehrungskolonie 4. Diese füllt den Hohlraum zu etwa 75% aus und umfasst in Form eines durchmischten Konglomerats eine vermehrungsfähige Population von Nützlingen 5 in Form von Raubmilben, eine vermehrungsfähige Population von Wirten 6 in Form von Opfer- bzw. Futtermilben sowie einen - auf die Art der Wirte abgestimmten - Nahrungsvorrat 7 für die Opfer- bzw. Futtermilben in Form verschiedener Getreideprodukte und sonstiger Zusätze. Die Nützlingspopulation umfasst dabei Nützlinge und die Wirtspopulation umfasst Wirte in verschiedenen Entwicklungsstadien.

Die Vermehrungs-, Brut- und Aufzuchtkugel 2 weist insgesamt vier Austrittsöffnungen 8 auf, die jeweils paarweise einander benachbart auf gegenüberliegenden Seiten der Vermehrungs-, Brut- und Aufzuchtkugel 2 angeordnet sind.

Das in Fig. 2 in einer Anwendung an einer (schematisch angedeuteten) Pflanze 9 gezeigte Ausbringsystem 10 für die Ausbringung von Nützlingen umfasst zwei Ausbringeinheiten 1 nach Fig. 1, die über eine etwa 20cm lange, aus einem biologisch abbaubaren Material bestehende Verbindungsschnur 11 miteinander verbunden sind. Ersichtlich ist eine einfache und doch dauerhafte Anbringung der beiden Ausbringeinheiten 1 an der Pflanze 9 möglich. An den beiden Ausbringeinheiten 1 sind noch Verbindungsschnurenden 12 zu erkennen, die davon herrühren, dass das Ausbringsystem 10 gemäß Fig. 2 aus einer Unterteilung eines längeren perlenschnurartigen Ausbringsystems in einzelne jeweils ein Paar von Ausbringeinheiten umfassende Gebilde nach Fig. 2 hervorgegangen ist.

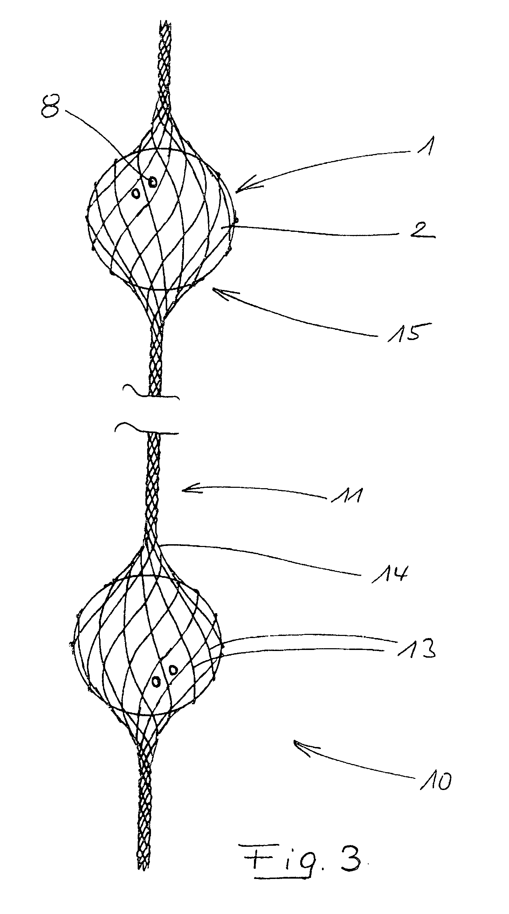

Fig. 3 veranschaulicht, dass die Verbindungsschnur 11, mittels welcher im Rahmen eines Ausbringsystems 10 jeweils zwei Ausbringeinheiten 1 der in Fig. 1 gezeigten Art miteinander verbunden sind, durch einen aus Einzelfäden 13 geflochtenen aufweitbaren Schlauch 14 gebildet sein kann. In diesem sind in entsprechend geweiteten Abschnitten 15 die Vermehrungs-, Brut- und Aufzuchtkugeln 2 aufgenommen.

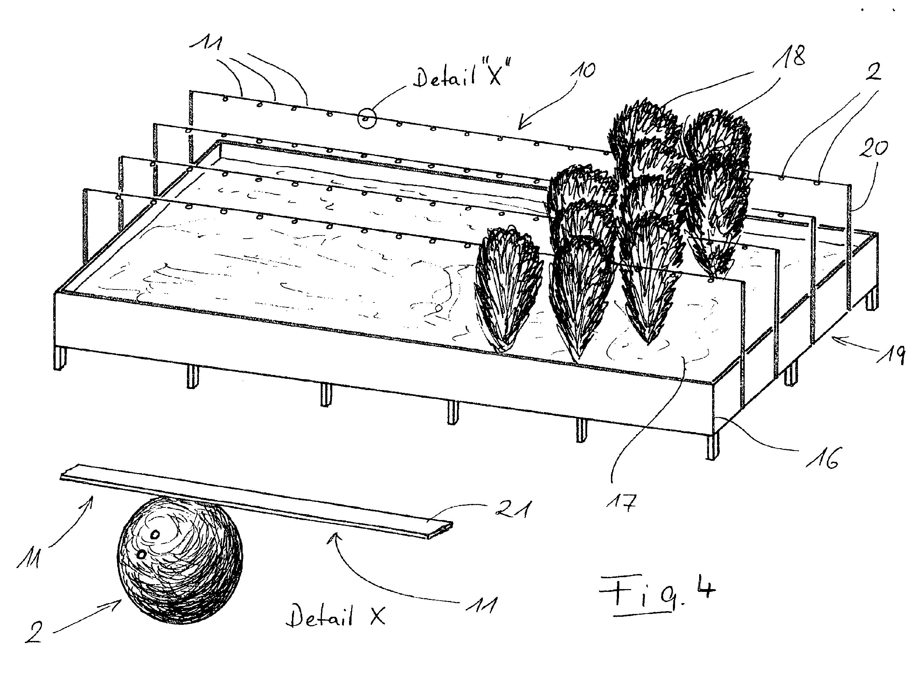

Fig. 4 zeigt die Anwendung des erfindungsgemäßen Ausbringsystems an einem Pflanztisch 16, welcher ein Pflanzbett 17 aus Pflanzerde aufnimmt, in der - in fünf Reihen - Nutzpflanzen 18 wachsen. An den beiden einander gegenüberliegenden Enden 19 des Pflanztisches 16 sind jeweils vier aufragende Spannstangen 20 vorgesehen. An diesen sind, jeweils in wahlweise veränderbarer Höhe, perlenschnurähnliche Ausbringsysteme 10 mit einer Mehrzahl von mittels Verbindungsschnüren 11 miteinander verbundenen Vermehrungs-, Brut- und Aufzuchtkugeln 2 angebracht. Die Ausbringsysteme erstrecken sich dabei zwischen jeweils zwei benachbarten Reihen der Nutzpflanzen 18 in einer auf deren Wuchshöhe abgestimmten Höhe über dem Pflanzbett 17. Die jeweils zwei Vermehrungs-, Brut- und Aufzuchtkugeln 2 miteinander verbindenden Verbindungsschnüre 11 gehen dabei (vgl. Detail "X") in dem Sinne ineinander über, dass sie eine durchgehende, sich über die Länge des Pflanztisches 16 erstreckenden Schnur 21 bilden, an der die einzelnen Vermehrungs-, Brut- und Aufzuchtkugeln 2 in bestimmten Abständen durch Kleben angebracht sind. Die Schnur 21 weist bei dem gezeigten Ausführungsbeispiel einen flachen Querschnitt auf.

## Patentansprüche

1. Ausbringsystem (10) für die Ausbringung mehrerer Nützlings-Vermehrungskolonien (4) in einen land- bzw. gartenwirtschaftlichen Bestand, umfassend eine Mehrzahl von der Ausbringung jeweils einer Nützlings-Vermehrungskolonie (4) in einen land- bzw. gartenwirtschaftlichen Bestand dienenden Ausbringeinheiten (1), mit den folgenden Merkmalen:
die Ausbringeinheiten (1) umfassen jeweils eine Vermehrungs-, Brut- und Aufzuchtkugel (2) für die Nützlinge;
die Vermehrungs-, Brut- und Aufzuchtkugeln (2) enthalten jeweils eine Nützlings-/Wirt-Population umfassend eine vermehrungsfähige Population von Nützlingen (5) sowie eine vermehrungsfähige Population von Wirten (6);
die Vermehrungs-, Brut- und Aufzuchtkugeln (2) enthalten weiterhin jeweils einen Nahrungsvorrat (7) für die Wirte;
sie bestehen aus einem biologisch abbaubaren Material; die Ausbringeinheiten sind mittels Verbindungsschnüren (11) miteinander verbunden.

2. Ausbringsystem nach Anspruch 1, **dadurch gekennzeichnet,**
**dass** die Vermehrungs-, Brut- und Aufzuchtkugeln (2) eine wasserabweisende Beschichtung aufweisen oder aus in Paraffin getränktem Zellstoff bestehen.

3. Ausbringsystem nach Anspruch 2, **dadurch gekennzeichnet,**
**dass** die wasserabweisende Beschichtung aus Paraffin besteht.

4. Ausbringsystem nach einem der Ansprüche 1 bis 3,
**dadurch gekennzeichnet,**
**dass** die die Vermehrungs-, Brut- und Aufzuchtkugeln (2) einen kapillaren Wasserspeicher umfassen.

5. Ausbringsystem nach einem der Ansprüche 1 bis 4,
**dadurch gekennzeichnet,**
**dass** die die Vermehrungs-, Brut- und Aufzuchtkugeln (2) auf jeder Hälfte zwei Austrittsöffnungen (8) besitzen.

6. Ausbringsystem nach einem der Ansprüche 1 bis 5,
**dadurch gekennzeichnet,**
**dass** die die Vermehrungs-, Brut- und Aufzuchtkugeln (2) einen Durchmesser zwischen 1,5cm und 2,5cm und/oder eine Wandstärke zwischen 0,5mm und 2mm aufweisen.

7. Ausbringsystem nach einem der Ansprüche 1 bis 6,
**dadurch gekennzeichnet,**
**dass** die Nützlingspopulationen Nützlinge (5) und/oder die Wirtspopulation Wirte (6) in verschiedenen Entwicklungsstadien umfassen.

8. Ausbringsystem nach einem der Ansprüche 1 bis 7,
**dadurch gekennzeichnet,**
**dass** die Verbindungsschnüre (11) aus einem biologisch abbaubaren Material bestehen.

9. Ausbringsystem nach einem der Ansprüche 1 bis 8, **dadurch gekennzeichnet,**
**dass** die Verbindungsschnüre (11) eine Reißfestigkeit zwischen dem 50-fachen und dem 500-fachen Wert der dem Gewicht einer Vermehrungs-, Brut- und Aufzuchtkugeln (2) entsprechenden Gewichtskraft aufweisen.

10. Ausbringsystem nach einem der Ansprüche 1 bis 9,
**dadurch gekennzeichnet,**
**dass** die Länge der Verbindungsschnüre (11) etwa dem 5-fachen bis 20-fachen Wert des Durchmessers der Vermehrungs-, Brut- und Aufzuchtkugeln (2) entspricht und/oder die Verbindungsschnüre einen gegenüber dem Durchmesser der Vermehrungs-, Brut- und Aufzuchtkugeln wesentlich kleinere Dicke aufweisen.

11. Ausbringsystem nach einem der Ansprüche 1 bis 10,
**dadurch gekennzeichnet,**
**dass** die Verbindungsschnüre (11) durch einen aufweitbaren Schlauch (14) gebildet sind, in dem in entsprechend aufgeweiteten Abschnitten (15) die Vermehrungs-, Brut- und Aufzuchtkugeln (2) aufgenommen sind.

12. Ausbringsystem nach einem der Ansprüche 1 bis 11,
**dadurch gekennzeichnet,**
**dass** die Verbindungsschnüre (11) eine durchgehende Schnur (21) bilden, an der die Vermehrungs-, Brut- und Aufzuchtkugeln (2) in definierten Abständen angebracht sind.

13. Ausbringsystem nach Anspruch 12,
**dadurch gekennzeichnet,**
**dass** es mehrere durchgehende Schnüre (21) aufweist.

14. Ausbringsystem nach einem der Ansprüche 1 bis 13,
**dadurch gekennzeichnet,**
**dass** die Verbindungsschnüre (11) einen flachen Querschnitt aufweisen.

15. Ausbringsystem nach einem der Ansprüche 1 bis 14,
**dadurch gekennzeichnet,**
**dass** die Vermehrungs-, Brut- und Aufzuchtkugeln (2) aus einem auf Zellstoff basierenden biologisch abbaubaren Material bestehen.

## Claims

1. A distribution system (10) for the distribution of a plurality of beneficial-organism breeding colonies (4) into an agricultural or horticultural crop, comprising a plurality of distribution units (1) used for distributing one beneficial-organism breeding colony (4) into an agricultural or horticultural crop in each case, with the following features:
the distribution units (1) comprise a breeding, hatching and rearing ball (2) for the beneficial organisms in each case;
the breeding, hatching and rearing balls (2) in each case contain a beneficial-organism/host population comprising a breeding-capable population of beneficial organisms (5) and also a breeding-capable population of hosts (6);
the breeding, hatching and rearing balls (2) furthermore contain a nutrient supply (7) for the hosts in each case;
they consist of a biodegradable material;
the distribution units are connected to one another by means of connecting twines (11).

2. The distribution system according to Claim 1, **characterised in that** the breeding, hatching and rearing balls (2) have a water-repellent coating or consist of cellulose soaked in paraffin.

3. The distribution system according to Claim 2, **characterised in that** the water-repellent coating consists of paraffin.

4. The distribution system according to one of Claims 1 to 3, **characterised in that** the breeding, hatching and rearing balls (2) comprise a capillary water storage system.

5. The distribution system according to one of Claims 1 to 4, **characterised in that** the breeding, hatching and rearing balls (2) have two outlet openings (8) on each half.

6. The distribution system according to one of Claims 1 to 5, **characterised in that** the breeding, hatching and rearing balls (2) have a diameter between 1.5cm and 2.5cm and/or a wall thickness between 0.5mm and 2mm.

7. The distribution system according to one of Claims 1 to 6, **characterised in that** the beneficial-organism population comprises beneficial organisms (5) and/or the host population comprises hosts (6) in various developmental stages.

8. The distribution system according to one of Claims 1 to 7, **characterised in that** the connecting twines (11) consist of a biologically degradable material.

9. The distribution system according to one of Claims 1 to 8, **characterised in that** the connecting twines (11) have a tear strength between 50-times and 500-times the value of the weight force corresponding to the weight of a breeding, hatching and rearing ball (2).

10. The distribution system according to one of Claims 1 to 9, **characterised in that** the length of the connecting twines (11) approximately corresponds to 5-times to 20-times the value of the diameter of the breeding, hatching and rearing balls (2) and/or the connecting twines have a substantially smaller thickness compared to the diameter of the breeding, hatching and rearing balls.

11. The distribution system according to one of Claims 1 to 10, **characterised in that** the connecting twines (11) are formed by an expandable tube (14), in which the breeding, hatching and rearing balls (2) are accommodated in correspondingly widened sections (15).

12. The distribution system according to one of Claims 1 to 11, **characterised in that** the connecting twines (11) form a continuous twine (21), to which the breeding, hatching and rearing balls (2) are attached at defined spacings.

13. The distribution system according to Claim 12, **characterised in that** it has a plurality of continuous twines (21).

14. The distribution system according to one of Claims 1 to 13, **characterised in that** the connecting twines (11) have a flat cross section.

15. The distribution system according to one of Claims 1 to 14, **characterised in that** the breeding, hatching and rearing balls (2) consist of a biologically degradable material based on cellulose.

## Revendications

1. Système d'épandage (10) pour l'épandage de plusieurs colonies de multiplication d'auxiliaires (4) sur un terrain agricole ou horticole, comprenant une pluralité d'unités d'épandage (1) servant à l'épandage d'une colonie respective de multiplication d'auxiliaires (4) sur un terrain agricole ou horticole, avec les caractéristiques suivantes :
les unités d'épandage (1) comprennent respectivement une boule de multiplication, de couvaison et d'élevage (2) pour les auxiliaires ;
les boules de multiplication, de couvaison et d'élevage (2) contiennent respectivement une population d'auxiliaires/hôtes comprenant une population réplicable d'auxiliaires (5) ainsi qu'une population réplicable d'hôtes (6) ;
les boules de multiplication, de couvaison et d'élevage (2) contiennent en outre respectivement une réserve de nourriture (7) pour les hôtes ;
elles sont constituées d'un matériau biodégradable ;
les unités d'épandage sont reliées entre elles au moyen de cordons de liaison (11).

2. Système d'épandage selon la revendication 1, **caractérisé en ce que** les boules de multiplication, de couvaison et d'élevage (2) comportent un revêtement hydrofuge ou sont constituées de pâte imprégnée de paraffine.

3. Système d'épandage selon la revendication 2, **caractérisé en ce que** le revêtement hydrofuge est constitué de paraffine.

4. Système d'épandage selon l'une des revendications 1 à 3, **caractérisé en ce que** les boules de multiplication, de couvaison et d'élevage (2) comprennent un réservoir d'eau capillaire.

5. Système d'épandage selon l'une des revendications 1 à 4, **caractérisé en ce que** les boules de multiplication, de couvaison et d'élevage (2) possèdent deux ouvertures de sortie (8) dans chaque moitié.

6. Système d'épandage selon l'une des revendications 1 à 5, **caractérisé en ce que** les boules de multiplication, de couvaison et d'élevage (2) présentent un diamètre entre 1,5 cm et 2,5 cm et/ou une épaisseur de paroi entre 0,5 mm et 2 mm.

7. Système d'épandage selon l'une des revendications 1 à 6, **caractérisé en ce que** les populations d'auxiliaires comprennent des auxiliaires (5) et/ou la population d'hôtes comprend des hôtes (6) à différents stades de développement.

8. Système d'épandage selon l'une des revendications 1 à 7, **caractérisé en ce que** les cordons de liaison (11) sont constitués d'un matériau biodégradable.

9. Système d'épandage selon l'une des revendications 1 à 8, **caractérisé en ce que** les cordons de liaison (11) présentent une résistance à la rupture entre 50 fois et 500 fois la valeur de la force pondérale correspondant au poids d'une boule de multiplication, de couvaison et d'élevage (2).

10. Système d'épandage selon l'une des revendications 1 à 9, **caractérisé en ce que** la longueur des cordons de liaison (11) mesure environ entre 5 fois et 20 fois la valeur du diamètre des boules de multiplication, de couvaison et d'élevage (2), et/ou **en ce que** les cordons de liaison (11) présentent une épaisseur nettement inférieure par rapport au diamètre des boules de multiplication, de couvaison et d'élevage.

11. Système d'épandage selon l'une des revendications 1 à 10, **caractérisé en ce que** les cordons de liaison (11) sont formés par un tuyau dilatable (14), dans lequel sont reçues les boules de multiplication, de couvaison et d'élevage (2) dans des sections dilatées (15) de façon correspondante.

12. Système d'épandage selon l'une des revendications 1 à 11, **caractérisé en ce que** les cordons de liaison (11) forment un cordon continu (21), sur lequel sont fixées les boules de multiplication, de couvaison et d'élevage (2) par intervalles définis.

13. Système d'épandage selon la revendication 12, **caractérisé en ce qu'**il comporte plusieurs cordons continus (21).

14. Système d'épandage selon l'une des revendications 1 à 13, **caractérisé en ce que** les cordons de liaison (11) présentent une section transversale plate.

15. Système d'épandage selon l'une des revendications 1 à 14, **caractérisé en ce que** les boules de multiplication, de couvaison et d'élevage (2) sont constituées d'un matériau biodégradable à base de pâte.
